# FASCICULE DE BREVET EUROPEEN

(11) **EP 2 515 910 B1**
(45) Date de publication et mention de la délivrance du brevet: **30.10.2013**
(21) Numéro de dépôt: 10808931.9
(22) Date de dépôt: 23.12.2010
(51) Int. Cl.: A61K 31/55, A61K 31/715, A61K 31/718, A61K 45/06, A61P 25/00, A61P 25/22, A61P 25/24, A61P 25/08

(54) **UTILISATION DE POLYSACCHARIDES DANS LE TRAITEMENT DU STRESS ET DE L'ANXIÉTÉ**
VERWENDUNG VON POLYSACCHARIDEN ZUR BEHANDLUNG VON STRESS UND VON ANGSTZUSTÄNDEN
USE OF POLYSACCHARIDES IN THE TREATMENT OF STRESS AND ANXIETY

(30) Priorité: 24.12.2009 FR 0959605
(43) Date de publication de la demande: 31.10.2012
(73) Titulaire: Roquette Freres, 62136 Lestrem (FR)
(72) Inventeur: GUERIN-DEREMAUX, Laëtitia, F-59850 Nieppe (FR); WILS, Daniel, F-59190 Morbecque (FR)
(74) Mandataire: Cabinet Plasseraud
(86) Numéro de dépôt international: PCT/FR2010/052911
(87) Numéro de publication internationale: WO 2011/077063

(56) Documents cités:
- EP-A1- 1 006 128
- EP-A1- 2 179 727
- WO-A1-02/074104
- WO-A2-2007/058923
- CN-A- 1 876 003
- CN-A- 101 077 357
- US-A- 5 760 014
- US-A1- 2006 084 629
- US-A1- 2006 147 569
- LI YUN FENG ET AL: "Inhibition of the oligosaccharides extracted from Morinda officinalis, a Chinese traditional herbal medicine, on the corticosterone induced apoptosis in PC12 cells.", LIFE SCIENCES, vol. 72, no. 8, 10 janvier 2003 (2003-01-10), pages 933-942, XP002585872, ISSN: 0024-3205

## Description

La présente invention porte sur l'utilisation d'un polysaccharide pour réduire le stress et l'anxiété chez l'homme ou chez l'animal.

La présente invention concerne également une composition contenant au moins un polysaccharide et au moins un agent actif pour son utilisation dans le traitement du stress, de l'anxiété ou des comportements dépressifs, des troubles du sommeil, des troubles obsessionnels compulsifs, de la boulimie, et de l'épilepsie chez l'homme ou l'animal.

Le stress, l'anxiété ou les comportements dépressifs voire suicidaires, les troubles du sommeil, les troubles obsessionnels compulsifs, la boulimie, la prise en charge thérapeutique de la douleur chronique ou de l'épilepsie font généralement l'objet de traitements à base d'antidépresseurs. Les antidépresseurs sont classés en quatre grandes familles: les inhibiteurs spécifiques de la recapture de sérotonine (ISRS), les tricycliques et tétracycliques, les inhibiteurs de la monoamine oxydase (IMAO) et les antidépresseurs atypiques.

Ces molécules sont généralement connues pour leurs nombreux effets secondaires qui sont responsables, dans une majorité des cas, d'une interruption du traitement. Parmi ces effets secondaires, on peut citer notamment les syndromes somatiques et gastro-intestinaux, des troubles du sommeil, des mouvements anormaux et des troubles du comportement, de la confusion mentale, des tremblements des extrémités ou des risques épileptogènes. En outre, ces molécules sont majoritairement connues pour l'addiction qu'elles entrainent.

US2006/147569 A1 décrit des compositions pour le traitement de la dépression et des symptômes associés avec une combinaison de *Psoralea coryfolia L.* et de *Poria cocos* (Schw.).

Li Yun Feng et al.,: "Inhibition of the oligosaccharides extracted from Morinda officinalis, a Chinese traditional herbal medecine, on the corticosterone induced apoptosis in PC12 cells. Life Sciences, vol. 72, no 8, 10 janvier 2003, 933-942 décrit l'utilisation d'inuline issue de *M. officinalis* dans le traitement de stress et de la dépression.

Afin de réduire la prise de ces psychotropes ou de remplacer totalement leur prise dans les cas les moins graves par un élément plus naturel ne comportant pas de risques majeurs de dépendance ou d'effets secondaires, la présente invention porte sur une composition comprenant au moins un polysaccharide et au moins un agent actif, utilisable dans le traitement du stress, de l'anxiété ou des comportements dépressifs, des troubles du sommeil, des troubles obsessionnels compulsifs, de la boulimie, et de l'épilepsie chez l'homme ou l'animal.

La présente invention vise également l'utilisation de ladite composition pour la préparation d'un médicament destiné à la prévention ou le traitement du stress, de l'anxiété ou des comportements dépressifs, des troubles du sommeil, des troubles obsessionnels compulsifs, de la boulimie, et de l'épilepsie chez l'homme ou l'animal.

Par le terme général de « polysaccharides », on entend des polymères formés d'un certain nombre de monosaccharides. Parmi ces polysaccharides, on distingue les homopolysaccharides constitués du même monosaccharide, et les hétéropolysaccharides formés de différents monosaccharides.

Le polysaccharide utilisé conformément à la présente invention présente :
- entre 15 et 50% de liaisons glucosidiques 1-6, préférentiellement entre 22 % et 45 %, plus préférentiellement 20 et 40%, et encore plus préférentiellement entre 25 et 35 %,
- une teneur en sucres réducteurs inférieure à 20%, préférentiellement comprise entre 2 et 20%, plus préférentiellement entre 2,5 et 15%, et encore plus préférentiellement entre 3,5 et 10%,
- un indice de polymolécularité inférieur à 5, préférentiellement compris entre 1 et 4, plus préférentiellement entre 1,5 et 3, et
- une masse moléculaire moyenne en nombre Mn inférieure à 4500 g/mole, préférentiellement comprise entre 400 et 4500 g/mole, plus préférentiellement entre 500 et 3000 g/mole, de préférence entre 700 et 2800 g/mole, et encore plus préférentiellement comprise entre 1000 et 2600 g/mole.

Le polysaccharide utilisé conformément à l'invention est soluble dans l'eau.

Il peut être préparé selon le procédé décrit dans le brevet EP 1 006 128. Ce brevet décrit l'obtention d'une maltodextrine branchée qui se distingue des maltodextrines standards notamment par leur richesse en liaison glucosidiques 1 -> 6.

De façon préférentielle, le polysaccharide selon l'invention est un amidon modifié obtenu par une hydrolyse acide ou enzymatique optionnellement suivie d'un traitement à la chaleur. Typiquement, le polysaccharide est une dextrine branchée ou une maltodextrine branchée.

Au sens de l'invention, on entend par « maltodextrines branchées », des maltodextrines dont la teneur en liaisons glucosidiques 1 -> 6 est supérieure à celle des maltodextrines standards. Ainsi, les maltodextrines standards se définissent comme des mélanges purifiés et concentrés de glucose et de polymères de glucose essentiellement liés en 1 -> 4 avec seulement de 4 à 5 % de liaisons glucosidiques 1 - > 6, de poids moléculaires extrêmement variés, complètement solubles dans l'eau et à faible pouvoir réducteur.

Les maltodextrines standards sont classiquement produites par hydrolyse acide ou enzymatique d'amidon. La classification des maltodextrines standards repose principalement sur la mesure de leur pouvoir réducteur, exprimé classiquement par la notion de Dextrose Equivalent (D.E.). Sur ce point particulier, la définition des maltodextrines reprise dans les Monograph Spécifications du Food Chemical Codex précise que la valeur de D.E. ne doit pas excéder 20.

La mesure du D.E. ne donne en fait qu'une idée approximative du Degré de Polymérisation (D.P.) moyen du mélange du glucose et des polymères de glucose constitutifs des maltodextrines standards et donc de leur masse moléculaire moyenne en nombre (Mn). Pour compléter la caractérisation de la distribution des masses moléculaires des maltodextrines standards, la détermination d'un autre paramètre est importante, celui de la masse moléculaire moyenne en poids (Mp).

Dans la pratique, les valeurs de Mn et de Mp se mesurent par différentes techniques. On utilise par exemple une méthode de mesure adaptée aux polymères de glucose, qui repose sur la chromatographie de perméation de gel sur des colonnes de chromatographie étalonnées avec des pullulans de masses moléculaires connues.

Le rapport Mp/Mn est appelé indice de polymolécularité (I.P.) et permet de caractériser globalement la distribution des masses moléculaires d'un mélange polymérique. En règle générale, la répartition en masses moléculaires des maltodextrines standards conduit à des I.P. compris entre 5 et 10.

Le brevet EP 1 006 128 décrit une maltodextrine branchée selon l'invention obtenue par la mise en oeuvre des étapes suivantes :
a. préparation d'un amidon acidifié déshydraté présentant une humidité inférieure à 5 %, de préférence inférieure ou égale à 4 %,
b. traitement de l'amidon acidifié ainsi déshydraté dans un réacteur de type à couche mince à une température comprise entre 120 et 300°C, de préférence comprise entre 150 et 200°C.
c. recueille, purification et de préférence concentration des dérivés d'amidon branchés ainsi obtenus,
d. fractionnement moléculaire desdits dérivés d'amidon branchés en fonction de leur masse moléculaire moyenne en nombre de manière à obtenir les maltodextrines branchées.

Selon une variante, ledit polysaccharide présente un poids moléculaire Mw compris entre 1000 et 6000 g/mole, préférentiellement compris entre 1500 et 5000 g/mole, et plus préférentiellement compris entre 3000 et 5000 g/mole.

Selon une particularité de l'invention, ledit agent actif est un antidépresseur. Avantageusement, l'antidépresseur est choisi parmi les inhibiteurs spécifiques de la recapture de la sérotonine (ISRS), les tricycliques et tétracycliques, les inhibiteurs de la monoamine oxydase (IMAO), les antidépresseurs atypiques et leurs mélanges.

Parmi les actifs appropriés, sont préférés les antidépresseurs de la classe des benzodiazépines ou des inhibiteurs de la recapture de la sérotonine.

Ces compositions peuvent être formulées pour une administration aux mammifères, y compris l'homme. La posologie varie selon le traitement et selon l'affection en cause. Ces compositions sont réalisées de façon à pouvoir être administrées par la voie digestive, notamment pour une administration sublinguale, buccale ou rectale. Le mélange peut être administré sous formes unitaires d'administration, en mélange avec des supports pharmaceutiques classiques, aux animaux ou aux êtres humains.

Les formes unitaires d'administration appropriées comprennent les formes par voie orale telles que les comprimés, les gélules, les poudres, les granules et les solutions ou suspensions orales, ou les sirops, ou les formes par voie rectale tels les suppositoires.

Une préparation sous forme de sirop ou d'élixir peut contenir au moins un polysaccharide associé à l'agent actif conjointement avec un édulcorant, un antiseptique, ainsi qu'un agent donnant du goût et un colorant approprié.

Les poudres ou les granules dispersibles dans l'eau peuvent contenir au moins un polysaccharide selon l'invention en mélange avec l'ingrédient actif. Ils peuvent par ailleurs contenir différents agents, seuls ou en mélange, tels que des agents de dispersion, des agents mouillants, des agents de mise en suspension, des correcteurs du goût ou des édulcorants. Ces poudres peuvent être obtenues par atomisation ou broyage et les granules par granulation sèche ou humide ou co-atomisation d'un mélange selon l'invention.

Une préparation sous forme de comprimé peut être ou non suivie d'une étape d'enrobage permettant de contrôler la libération du mélange selon l'invention.

Le polysaccharide utilisé selon l'invention peut être employé en thérapie seul, ou en combinaison avec au moins un autre agent actif. Cet autre agent actif est en particulier choisi parmi les actifs appropriés pour le traitement de l'anxiété, des troubles du sommeil et de l'épilepsie, ou d'adjuvants permettant d'améliorer l'activité du mélange selon l'invention, ou encore d'autres actifs connus pour leur emploi dans le traitement desdites affections. De tels agents actifs sont bien connus de l'homme du métier et sont disponibles dans le commerce ou encore décrits dans des ouvrages de référence.

L'invention porte aussi sur l'utilisation d'un polysaccharide présentant :
- entre 15 et 50% de liaisons glucosidiques 1-6,
- une teneur en sucres réducteurs inférieure à 20%
- un indice de polymolécularité inférieur à 5,
- une masse moléculaire moyenne en nombre Mn inférieure à 4500 g/mole pour la préparation d'un médicament, destiné au traitement du stress, de l'anxiété ou des comportements dépressifs, des troubles du sommeil, des troubles obsessionnels compulsifs, de la boulimie et de l'épilepsie chez l'homme ou l'animal.

L'utilisation de polysaccharide dans le traitement de l'anxiété ou du stress permet l'utilisation d'une molécule d'origine naturelle sans effets secondaires tels que l'addiction.

L'invention vise une méthode pour fabriquer un médicament destiné au traitement et/ou à la prévention du stress, de l'anxiété ou des comportements dépressifs, des troubles du sommeil, des troubles obsessionnels compulsifs, de la boulimie, et de l'épilepsie chez l'homme ou l'animal, caractérisée en ce que un polysaccharide présentant :
- entre 15 et 50% de liaisons glucosidiques 1-6,
- une teneur en sucres réducteurs inférieure à 20%,
- un indice de polymolécularité inférieur à 5, et
- une masse moléculaire moyenne en nombre Mn inférieure à 4500 g/mole,
est utilisé, seul ou en mélange, avec au moins un agent actif dans le traitement du stress, de l'anxiété ou des comportements dépressifs, des troubles du sommeil, des troubles obsessionnels compulsifs, de la boulimie, et de l'épilepsie chez l'homme ou l'animal.

Le polysaccharide est généralement administré dans des proportions journalières de 1g à 100g et préférentiellement de 5g à 50g et plus préférentiellement encore de 8 à 40g.

L'invention se rapporte aussi à des compositions pharmaceutiques contenant, une quantité efficace d'au moins un agent actif dans le traitement du stress, de l'anxiété ou des comportements dépressifs, des troubles du sommeil, des troubles obsessionnels compulsifs, de la boulimie, et de l'épilepsie chez l'homme ou l'animal et d'au moins un polysaccharide défini ci-dessous, dans des rapports polysaccharide / agent actif compris entre 500 et 104, de préférence entre 300 et 105 et plus préférentiellement encore entre 200 et 106.

L'association d'un agent actif et dudit polysaccharide permet l'utilisation d'une plus faible quantité d'actif sans pour autant réduire l'action du mélange sur les symptômes de stress ou d'anxiété. Ce mélange peut être envisagé lors d'un processus d'arrêt progressif d'un traitement ou dans des cas où le bénéfice d'un traitement médicamenteux serait trop faible par rapport aux risques d'addiction encourus par le patient.

Un mode de réalisation de l'invention concerne un kit pour le traitement thérapeutique ou prophylactique du corps humain ou animal comprenant :
a) une première composition comprenant un polysaccharide tel que défini précédemment; et
b) une deuxième composition comprenant un agent actif dans le traitement du stress, de l'anxiété ou des comportements dépressifs, des troubles du sommeil, des troubles obsessionnels compulsifs, de la boulimie, et de l'épilepsie chez l'homme ou l'animal.

L'invention a également pour objet un médicament comprenant une quantité efficace d'un polysaccharide présentant :
- entre 15 et 50% de liaisons glucosidiques 1-6,
- une teneur en sucres réducteurs inférieure à 20%
- un indice de polymolécularité inférieur à 5,
- une masse moléculaire moyenne en nombre Mn inférieure à 4500 g/mole
et un véhicule pharmacologiquement acceptable pour le traitement du stress, de l'anxiété ou des comportements dépressifs, des troubles du sommeil, des troubles obsessionnels compulsifs, de la boulimie et de l'épilepsie chez l'homme ou l'animal.

Avantageusement, les compositions pharmaceutiques selon l'invention comprennent également un véhicule pharmaceutique approprié.

Typiquement l'invention vise une méthode de traitement et/ou de prévention du stress, de l'anxiété ou des comportements dépressifs, des troubles du sommeil, des troubles obsessionnels compulsifs, de la boulimie, et de l'épilepsie chez l'homme ou l'animal, comprenant une étape d'administration à un patient qui en a besoin d'une quantité pharmaceutiquement efficace d'un polysaccharide présentant :
- entre 15 et 50% de liaisons glucosidiques 1-6,
- une teneur en sucres réducteurs inférieure à 20%,
- un indice de polymolécularité inférieur à 5, et
- une masse moléculaire moyenne en nombre Mn inférieure à 4500 g/mole,
ledit polysaccharide étant administré, seul ou en mélange, avec au moins un agent actif dans le traitement du stress, de l'anxiété ou des comportements dépressifs, des troubles du sommeil, des troubles obsessionnels compulsifs, de la boulimie, et de l'épilepsie chez l'homme ou l'animal.

Selon un autre aspect de la présente invention, celle-ci se rapporte également à un complément alimentaire convenant pour supplémenter l'alimentation des personnes sujettes notamment à l'anxiété, aux troubles du sommeil et à l'épilepsie, contenant une quantité efficace d'un polysaccharide selon l'invention en combinaison avec des supports alimentaires de nature protéique ou glucidique.

L'invention va être maintenant décrite plus en détail dans les exemples ci-après, basés sur des études pharmaco-comportementales chez le rat Wistar.

### Exemple 1 :

Les effets d'un oligosaccharide administré par voie orale aux doses de 0,7 et 1,4g/kg pendant 14 jours, ont été évalués dans une Batterie Fonctionnelle d'Observation (FOB) chez le rat mâle Wistar. Ce test FOB a pour objectif d'évaluer l'évolution des paramètres comportementaux, neurologiques et physiologiques des rats en fonction des produits testés.

Pour cela, un polysaccharide (PS1) tel que défini dans le tableau 1 est administré par gavage oesophagique quotidiennement à ces 2 doses pendant 14 jours (6 rats par groupe). De même, un témoin véhicule constitué par de l'eau de source est administré dans ces conditions. Le témoin positif de référence est du diazépam : de J0 à J13, de la méthycellulose est administrée aux rats et à J14, le diazépam à 3mg/kg de poids corporel est également administré.

**Tableau 1**

| | PS1 |
|---|---|
| Mn (g/mole) | 2640 |
| Mw (g/mole) | 4941 |
| Mn/Mw | 1,9 |
| Liaisons 1-6 (%) | 29-32 |
| Sucres réducteurs (%) | 3,9 |

Les animaux subissent la batterie fonctionnelle d'observation avant traitement à J0 et en fin d'étude à J14.

Chaque test comporte 3 phases d'observation :
- une phase d'observation directe durant laquelle l'animal n'est pas dérangé,
- une phase d'observation active pendant laquelle l'animal est manipulé,
- une phase consacrée à l'évaluation des réactions des animaux à des tests de réactivité.

Plusieurs variables sont enregistrées :
- Effets comportementaux : activité locomotrice spontanée, troubles du comportement locomoteur, émotivité (fréquence des cris), réaction de fuite au toucher, irritabilité, comportements d'agressivité et de freezing provoqués, somnolence, miction et défécation, réponses sensorimotrices (placement visuel, pincement de l'orteil et réaction à un stimulus sonore).
- Effets neurologiques : réponse pupillaire, réflexe palpébral, élévation pelvienne, position de la queue, tonicité musculaire des membres et de l'abdomen, test de retournement, test d'agrippement, test de réaction antigravitaire, tremblements et piloérection.
- Effets physiologiques : salivation, lacrymation, diarrhée, température rectale, rythme cardiaque et respiratoire.

Les résultats montrent qu'avant le premier traitement, aucune différence significative n'a été mise en évidence lors de la première FOB.

Après 14 jours d'administration des produits, des différences significatives sont observées entre les rats des différents groupes.

| Scores | Véhicule (eau) | Référence (diazépam) | PS1 0,7g/kg | PS1 1,4g/kg |
|---|---|---|---|---|
| Activité spontanée à J14 | 1,68 | 2.16 | 1,49 | 1,16 |
| *p (vs. Véhicule)* | | *p<0,10* | *NS* | *p<0,10* |
| Réaction à l'approche d'un doigt à J14 | 0,84 | 0,18 | 0,18 | 0,00 |
| *p (vs. Véhicule)* | | *p<0,05* | *p<0,05* | *p<0,01* |
| Fréquence respiratoire à J14 | 2,00 | 1,14 | 1,49 | 0,98 |
| *p (vs. Véhicule)* | | *p<0,01* | *p<0,10* | *p<0,001* |
| Réaction de sursaut à un stimulus sonore à J14 | 1,50 | 0,00 | 0,33 | 0,16 |
| *p (vs. Véhicule)* | | *p<0,01* | *p<0,05* | *p<0,01* |

### Définition des scores :

- Activité spontanée : 0=excité ; 1 à 3=mouvements rapides modérés ; 4 : aucune activité
- Réaction à l'approche d'un doigt : 0=aucune ; 1 à 3= échappement de léger à fort
- Fréquence respiratoire : 0= lente ; 1=normale ; 2=rapide
- Réaction de sursaut à un stimulus sonore : 0=léger ; 1=<1 cm ; 2=>1 cm

### En comparaison du véhicule à J14 :

- Activité spontanée : Le test de Mann-Whitney montre que les rats du groupe référence tendent à être significativement moins actifs, alors que les rats du groupe PS1 0,7 g/kg et 1,4 g/kg tendent à être significativement plus actifs. Pour le groupe PS1, les rats montrent un intérêt pour l'environnement plus fort
- Réaction à l'approche d'un doigt : Le test de Mann-Whitney montre que les rats du groupe référence et PS1 à 0,7 g/kg et 1,4 g/kg sont significativement moins réactifs à l'approche d'un doigt. En outre, ces rats sont plus calmes, moins anxieux.
- Fréquence respiratoire : Le test de Mann-Whitney montre que les rats du groupe référence et PS1 à 1,4g/kg présentent des fréquences respiratoires, mais également cardiaques, significativement plus lentes. Pour les groupes PS1, les rats sont plus calmes.
- Réaction de sursaut à un stimulus sonore : Le test de Mann-Whitney montre que les rats de tous les groupes présentent des sursauts significativement moins importants. Les rats des groupes PS1 sont plus calmes.

### En comparaison de la référence à J14 :

- Activité spontanée : Le test de Mann-Whitney montre que les rats du groupe PS1 à 0,7 g/kg et 1,4 g/kg sont significativement plus actifs.
- Réaction à l'approche d'un doigt : Le test de Mann-Whitney ne montre pas de différence.
- Fréquence respiratoire : Le test de Mann-Whitney ne montre pas de différence.
- Réaction de sursaut à un stimulus sonore : Le test de Mann-Whitney ne montre pas de différence.

Pour conclure, l'ensemble des paramètres étudiés montrent que les rats des groupes traités au PS1, aux deux doses, ont été moins anxieux, plus curieux après 14 jours de traitement.

De plus, pour un certain nombre de critères, le traitement aux polysaccharides PS1 montre les mêmes effets que celui au diazépam.

### Exemple 2

Les effets anti-stress d'un polysaccharide PS1 administré par voie orale pendant 11 jours, ont été évalués chez le rat mâle Wistar dans un test de retournement.

Pour cela, le PS1 est administré par gavage oesophagique quotidiennement aux doses de 0,7 ; 1,4 et 2,8 g/kg de poids corporel pendant 11 jours (16 rats par groupe). De même, un témoin véhicule représenté par de l'eau de source est administré dans ces conditions.

Quotidiennement de J1 à J11, les rats subissent un test de retournement. Ce test consiste à positionner le rat sur la paume de la main sur le dos et d'évaluer sa vitesse de retournement. Un pourcentage de retournement lent est calculé par groupe de rats pour la période de traitement de J1 à J11 après attribution d'un score de 0 = retournement rapide ou 1 = retournement lent.

Les résultats obtenus sont présentés dans le tableau ci-dessous. Ils sont exprimés en nombre en % de rats se retournant plus lentement entre J1 et J11

| | *Véhicule (eau)* | *PS1* /*0,7g*/*kg* | *PS1* /*1,4g*/*kg* | *PS1* /*2,8g*/*kg* | |
|---|---|---|---|---|---|
| *Retournement à J0* | *0,00 ± 0,00* | *0,00 ± 0,00* | *0,00 ± 0,00* | *0,00 ± 0,00* | |
| *Retournement entre J1 et J11* | *17,71 ± 7,94* | *41,*67 *± 8,63* | *36,46 ± 6,67* | *24,48 ± 6,61* | |

Il existe un phénomène d'habituation des animaux au cours du temps : le pourcentage de rats se retournant lentement passe de 0% à 17% pour le lot témoin véhicule (eau). Par contre, ce pourcentage double pour les lots PS1 à 0,7 g et 1,4 g pour atteindre environ 36 à 41%. Les animaux sont donc moins stressés de se retrouver dans cette position.

### Exemple 3

Les effets anti-stress d'un polysaccharide PS1, administré par voie orale pendant 14 jours, ont été évalués chez le rat mâle Wistar dans le test d'Enfouissement Défensif Conditionné (EDC).

Pour cela, le PS1 est administré par gavage oesophagique quotidiennement aux doses de 0,7 ; 1,4 et 2,8 g/kg de poids corporel pendant 14 jours (8 rats par groupe). De même, un témoin véhicule représenté par de l'eau de source est administré dans les mêmes conditions. Le témoin positif de référence est du diazépam : de J0 à J13, de la méthycellulose est administrée aux rats et à J14, le diazépam à 1 mg/kg ou 3 mg/kg de poids corporel est administré aux rats. Le mélange PS1 et diazépam est également étudié : le PS1 est administré pendant 14 jours seul et à J14, le diazépam à 1 mg/kg est ajouté au gavage oesophagique de la PS1.

A J14, les rats subissent le test d'Enfouissement Défensif Conditionné (EDC). Pour cela, une sonde électrique est insérée dans une cage avant le début du test. Chaque rat est déposé dans le dispositif expérimental du côté opposé à la sonde et un choc électrique unique, de faible intensité (2 mA) est délivré à l'animal au moment où il pose une patte antérieure pour la première fois sur la sonde. Suite au choc, le comportement du rat est enregistré pendant 5 minutes ; un score global de stress est attribué pour chaque rat. Le score est d'autant plus élevé que le stress est important

Les résultats obtenus sont présentés dans le tableau ci-après.

| | Véhicule (eau) | PS1 / 0,7g/kg | PS1 / 1,4g/kg | PS1 / 2,8g/kg | Référence Diazépam 3mg/kg | Référence Diazépam 1mg/kg | PS1 2,8g/kg + Diazépam 1mg/kg |
|---|---|---|---|---|---|---|---|
| Moyenne ± ESM | 121,94 ± 14,70 | 117,06 ± 17,06 | 90,50 ± 17,17 | 107,63 ± 12,43 | 65,25 ± 7,15 | 92,57 ± 17,99 | 75,36 ± 12,72 |
| p (vs véhicule) | - | NS | Tendance | NS | p<0,01 | Tendance | p<0,05 |

- Les résultats statistiques montrent que les scores globaux de stress des rats des groupes Diazépam 3 mg/kg, et PS1 2,8 g/kg+Diazépam 1 mg/kg sont significativement inférieurs à celui du groupe véhicule.
- Les scores des groupes PS1 à 1,4 g/kg et Diazépam 1 mg/kg tendent à être inférieurs à celui du groupe véhicule.
- Les scores des groupes PS1 à 0,7 g/kg et 2,8 g/kg ne sont pas significativement différents de celui du groupe véhicule, bien que les scores soient inférieurs pour ces 2 lots.

Il est possible de conclure que :
- la dose intermédiaire de PS1 à 1,4 g/kg présente un résultat intéressant sur le score de stress.
- les effets observés pour les lots PS1 et Diazépam sont significativement identiques.
- individuellement, les scores des groupes Diazépam à 1 mg/kg et PS1 à 2,8 g/kg sont respectivement de 92 et 107. Lorsque ces 2 produits sont associés, le score global diminue jusqu'à 75. La PS1 potentialise les effets du Diazépam. Cette synergie permettrait de limiter les doses administrées de Diazépam et d'en limiter les effets secondaires. La consommation de PS1 va dans le sens d'une optimisation de l'effet d'un médicament.

## Revendications

1. Composition comprenant au moins un polysaccharide et au moins un agent actif pour son utilisation dans le traitement du stress, de l'anxiété ou des comportements dépressifs, des troubles du sommeil, des troubles obsessionnels compulsifs, de la boulimie, et de l'épilepsie chez l'homme ou l'animal, ledit polysaccharide présentant :
- entre 15 et 50 % de liaisons glucosidiques 1-6,
- une teneur en sucres réducteurs inférieure à 20%,
- un indice de polymolécularité inférieur à 5 et
- une masse moléculaire moyenne en nombre Mn inférieure à 4500 g/mole.

2. Composition selon la revendication 1, pour son utilisation selon la revendication 1, **caractérisée en ce que** ledit polysaccharide présente :
- entre 20 % et 40 % de liaisons glucosidiques 1-6,
- une teneur en sucres réducteurs comprise entre 2 et 20%,
- un indice de polymolécularité compris entre 1 et 4, et
- une masse moléculaire moyenne en nombre Mn comprise entre 500 et 3000 g/mole

3. Composition selon l'une ou l'autre des revendications 1 et 2, pour son utilisation selon l'une ou l'autre des revendications 1 et 2, **caractérisée en ce que** le polysaccharide présente un poids moléculaire Mw compris entre 1000 et 6000 g/mole, préférentiellement compris entre 1500 et 5000 g/mole, plus préférentiellement compris entre 3000 et 5000 g/mole.

4. Composition selon l'une quelconque des revendications 1 à 3, pour son utilisation selon l'une quelconque des revendications 1 à 3 **caracterisée en ce qu'**au moins un agent actif est un antidépresseur.

5. Composition selon la revendication 4, pour son utilisation selon la revendication 4 **caractérisée en ce que** ledit agent actif est choisi parmi les inhibiteurs spécifiques de la recapture de sérotonine (ISRS), les tricycliques et tétracycliques, les inhibiteurs de la monoamine oxydase (IMAO), les antidépresseurs atypiques et leurs mélanges.

6. Composition selon l'une quelconque des revendications 1 à 5, pour son utilisation selon l'une quelconque des revendications 1 à 5 **caractérisée en ce qu'**elle se présente sous forme d'administration sublinguale, buccale ou rectale.

7. Composition selon la revendication 6, pour son utilisation selon la revendication 6 **caractérisée en ce qu'**elle est sous forme de comprimés, de gélules, de poudres, de granules, de solutions ou suspensions orales, d'émulsions, de sirops ou de suppositoires.

8. Médicament comprenant une quantité efficace d'un polysaccharide présentant :
- entre 15 et 50% de liaisons glucosidiques 1-6,
- une teneur en sucres réducteurs inférieure à 20%
- un indice de polymolécularité inférieur à 5,
- une masse moléculaire moyenne en nombre Mn inférieure à 4500 g/mole
et un véhicule pharmacologiquement acceptable pour son utilisation dans le traitement du stress, de l'anxiété ou des comportements dépressifs, des troubles du sommeil, des troubles obsessionnels compulsifs, de la boulimie et de l'épilepsie chez l'homme ou l'animal

9. Utilisation d'au moins un polysaccharide présentant :
- entre 15 et 50 % de liaisons glucosidiques 1-6,
- une teneur en sucres réducteurs inférieure à 20%
- un indice de polymolécularité inférieur à 5, et
- une masse moléculaire moyenne en nombre Mn inférieure à 4500 g/mole
pour la préparation d'un médicament destiné au traitement du stress, de l'anxiété ou des comportements dépressifs, des troubles du sommeil, des troubles obsessionnels compulsifs, de la boulimie et de l'épilepsie chez l'homme ou l'animal.

10. Utilisation d'au moins un polysaccharide présentant :
- entre 15 et 50 % de liaisons glucosidiques 1-6,
- une teneur en sucres réducteurs inférieure à 20%
- un indice de polymolécularité inférieur à 5, et
- une masse moléculaire moyenne en nombre Mn inférieure à 4500 g/mole
et d'au moins un agent actif pour le traitement du stress, de l'anxiété ou des comportements dépressifs, des troubles du sommeil, des troubles obsessionnels compulsifs, de la boulimie et de l'épilepsie chez l'homme ou l'animal,
pour la préparation d'un médicament destiné au traitement du stress, de l'anxiété ou des comportements dépressifs, des troubles du sommeil, des troubles obsessionnels compulsifs, de la boulimie et de l'épilepsie chez l'homme ou l'animal.

11. Kit pour le traitement thérapeutique ou prophylactique du corps humain ou animal comprenant :
a) une première composition comprenant un polysaccharide présentant :
- entre 15 et 50% de liaisons glucosidiques 1-6,
- une teneur en sucres réducteurs inférieure à 20%
- un indice de polymolécularité inférieur à 5,
- une masse moléculaire moyenne en nombre Mn inférieure à 4500 g/mole; et
b) une deuxième composition comprenant un agent actif dans le traitement du stress, de l'anxiété ou des comportements dépressifs, des troubles du sommeil, des troubles obsessionnels compulsifs, de la boulimie, et de l'épilepsie chez l'homme ou l'animal.

## Patentansprüche

1. Zusammensetzung umfassend mindestens ein Polysaccharid und mindestens einen Wirkstoff zur Verwendung für die Behandlung von Stress, Angst oder depressivem Verhalten, Schlafstörungen, Zwangstörungen, Bulimie, und Epilepsie bei Menschen oder Tieren, wobei das Polysaccharid :
- zwischen 15 und 50% an 1-6 glukosidischen Bindungen,
- einen Gehalt an reduzierenden Zuckern von weniger als 20%,
- einen Polydispersitäts-Index von weniger als 5 und
- einen Zahlenmittelwert des Molekulargewichts Mn von weniger als 4500 g/mol
aufweist.

2. Zusammensetzung nach Anspruch 1 zur Verwendung nach Anspruch 1, **dadurch gekennzeichnet, dass** das Polysaccharid:
- zwischen 20 und 40% an 1-6 glukosidischen Bindungen,
- einen Gehalt an reduzierenden Zuckern zwischen 2 und 20%,
- einen Polydispersitäts-Index zwischen 1 und 4, und
- ein Zahlenmittelwert des Molekulargewichts Mn zwischen 500 und 3000 g/mol
aufweist.

3. Zusammensetzung nach einem der Ansprüche 1 oder 2 zur Verwendung nach einem der Ansprüche 1 oder 2, **dadurch gekennzeichnet, dass** das Polysaccharid ein Molekulargewicht Mw zwischen 1000 und 6000 g/mol, vorzugsweise zwischen 1500 und 5000 g/mol, mehr bevorzugt zwischen 3000 und 5000 g/mol aufweist.

4. Zusammensetzung nach einem der Ansprüche 1 bis 3 zur Verwendung nach einem der Ansprüche 1 bis 3, **dadurch gekennzeichnet, dass** der mindestens eine Wirkstoff ein Antidepressivum ist.

5. Zusammensetzung nach Anspruch 4 zur Verwendung nach Anspruch 4, **dadurch gekennzeichnet, dass** der Wirkstoff aus den spezifischen Wiederaufnahme-Inhibitoren von Serotonin (ISRS), den Trizyklen und den Tetrazyklen, den Monoamin-OxydaseInhibitoren (IMAO), den atypischen Antidepressiva und Mischungen davon ausgewählt ist.

6. Zusammensetzung nach einem der Ansprüche 1 bis 5 zur Verwendung nach einem der Ansprüche 1 bis 5, **dadurch gekennzeichnet, dass** sie eine sublinguale, bukkal oder rektale Verabreichungsform ist.

7. Zusammensetzung nach Anspruch 6 zur Verwendung nach Anspruch 6, **dadurch gekennzeichnet, dass** sie in Form von Tabletten, Kapseln, Pulvern, Kügelchen, Lösungen oder oralen Suspensionen, Emulsionen, Sirupen oder Suppositorien ist.

8. Medikament umfassend eine wirksame Menge eines Polysaccharids, welches :
- zwischen 15 und 50% an 1-6 glukosidischen Bindungen,
- einen Gehalt an reduzierenden Zuckern von weniger als 20%,
- einen Polydispersitäts-Index von weniger als 5 und
- einen Zahlenmittelwert des Molekulargewichts Mn von weniger als 4500 g/mol
und einen pharmazeutisch akzeptablen Träger aufweist zur Behandlung von Stress, Angst oder depressivem Verhalten, Schlafstörungen, Zwangstörungen, Bulimie, und Epilepsie bei Menschen oder Tieren.

9. Verwendung von mindestens einem Polysaccharid, welches :
- zwischen 15 und 50% an 1-6 glukosidischen Bindungen,
- einen Gehalt an reduzierenden Zuckern von weniger als 20%,
- einen Polydispersitäts-Index von weniger als 5 und
- einen Zahlenmittelwert des Molekulargewichts Mn von weniger als 4500 g/mol aufweist
zur Herstellung eines Medikaments zur Behandlung von Stress, Angst oder depressivem Verhalten, Schlafstörungen, Zwangstörungen, Bulimie, und Epilepsie bei Menschen oder Tieren.

10. Verwendung von mindestens einem Polysaccharid, welches :
- zwischen 15 und 50% an 1-6 glukosidischen Bindungen,
- einen Gehalt an reduzierenden Zuckern von weniger als 20%,
- einen Polydispersitäts-Index von weniger als 5 und
- einen Zahlenmittelwert des Molekulargewichts Mn von weniger als 4500 g/mol aufweist
und mindestens einen wirksamen Wirkstoff zur Behandlung von Stress, Angst oder depressivem Verhalten, Schlafstörungen, Zwangstörungen, Bulimie, und Epilepsie bei Menschen oder Tieren zur Herstellung eines Medikaments zur Behandlung von Stress, Angst oder depressivem Verhalten, Schlafstörungen, Zwangstörungen, Bulimie, und Epilepsie bei Menschen oder Tieren.

11. Kit zur therapeutischen oder profilaktischen Behandlung des menschlichen oder tierischen Körpers umfassend :
a) eine erste Zusammensetzung umfassend ein Polysaccharid, welches:
- zwischen 15 und 50% an 1-6 glukosidischen Bindungen,
- einen Gehalt an reduzierenden Zuckern von weniger als 20%,
- einen Polydispersitäts-Index von weniger als 5 und
- einen Zahlenmittelwert des Molekulargewichts Mn von weniger als 4500 g/mol aufweist; und
b) eine zweite Zusammensetzung umfassend einen Wirkstoff zur Behandlung von Stress, Angst oder depressivem Verhalten, Schlafstörungen, Zwangstörungen, Bulimie, und Epilepsie bei Menschen oder Tieren.

## Claims

1. A composition comprising at least one polysaccharide and at least one active agent for use in treating stress, anxiety or depressive behavior, sleep disorders, obsessive-compulsive disorders, bulimia and epilepsy in humans or animals, said polysaccharide having:
- between 15 and 50% of 1-6 glucosidic linkages,
- a reducing sugar content of less than 20%,
- a polydispersity index of less than 5, and
- a number-average molecular weight Mn of less than 4500 g/mol.

2. The composition as claimed in claim 1, for use according to claim 1 **characterized in that** said polysaccharide has:
- between 20% and 40% of 1-6 glucosidic linkages,
- a reducing sugar content of between 2 and 20%,
- a polydispersity index of between 1 and 4, and
- a number-average molecular weight Mn of between 500 and 3000 g/mol.

3. The composition as claimed in either of claims 1 and 2, for use according to any one of claim 1 or 2 **characterized in that** the polysaccharide has a molecular weight Mw of between 1000 and 6000 g/mol, preferentially between 1500 and 5000 g/mol, more preferentially between 3000 and 5000 g/mol.

4. The composition as claimed in any one of claims 1 to 3, for use according to any one of claims 1 to 3 **characterized in that** at least one active agent is an antidepressant.

5. The composition as claimed in claim 4, for use according to claim 4 **characterized in that** said active agent is chosen from selective serotonin reuptake inhibitors (SSRIs), tricyclic and tetracyclic antidepressants, monoamine oxidase inhibitors (MAOIs), atypical antidepressants, and mixtures thereof.

6. The composition as claimed in any one of claims 1 to 5, for use according to any one of claims 1 to 5 **characterized in that** it is in sublingual, oral or rectal administration form.

7. The composition as claimed in claim 6, for use according to claim 6 **characterized in that** it is in the form of tablets, gel capsules, powders, granules, oral solutions or suspensions, emulsions, syrups or suppositories.

8. A medicament comprising an effective amount of a polysaccharide having:
- between 15 and 50% of 1-6 glucosidic linkages,
- a reducing sugar content of less than 20%,
- a polydispersity index of less than 5,
- a number-average molecular weight Mn of less than 4500 g/mol,
and a pharmacologically acceptable vehicle for use in the treatment of stress, anxiety or depressive behavior, sleep disorders, obsessive-compulsive disorders, bulimia and epilepsy in humans or animals.

9. The use of at least one polysaccharide having:
- between 15 and 50% of 1-6 glucosidic linkages,
- a reducing sugar content of less than 20%,
- a polydispersity index of less than 5, and
- a number-average molecular weight Mn of less than 4500 g/mol,
for preparing a medicament intended for the treatment of stress, anxiety or depressive behavior, sleep disorders, obsessive-compulsive disorders, bulimia and epilepsy in humans or animals.

10. The use of at least one polysaccharide having:
- between 15 and 50% of 1-6 glucosidic linkages,
- a reducing sugar content of less than 20%,
- a polydispersity index of less than 5, and
- a number-average molecular weight Mn of less than 4500 g/mol,
and of at least one active agent for treating stress, anxiety or depressive behavior, sleep disorders, obsessive-compulsive disorders, bulimia and epilepsy in humans or animals,
for preparing a medicament intended for the treatment of stress, anxiety or depressive behavior, sleep disorders, obsessive-compulsive disorders, bulimia and epilepsy in humans or animals.

11. A kit for therapeutic or prophylactic treatment of the human or animal body, comprising:
a) a first composition comprising a polysaccharide having:
- between 15 and 50% of 1-6 glucosidic linkages,
- a reducing sugar content of less than 20%,
- a polydispersity index of less than 5,
- a number-average molecular weight Mn of less than 4500 g/mol; and
b) a second composition comprising an active agent for treating stress, anxiety or depressive behavior, sleep disorders, obsessive-compulsive disorders, bulimia and epilepsy in humans or animals.
